# EUROPEAN PATENT SPECIFICATION

(11) **EP 1 240 129 B1**
(45) Date of publication and mention of the grant of the patent: **04.08.2004**
(21) Application number: 00980851.0
(22) Date of filing: 29.11.2000
(51) Int. Cl.: C07C 51/235, C07C 53/00, C07C 53/126, C07C 53/128

(54) **OXIDATION PROCESS**
OXIDATIONSVERFAHREN
PROCEDE D'OXYDATION

(30) Priority: 22.12.1999 US 470361
(43) Date of publication of application: 18.09.2002
(73) Proprietor: Celanese International Corporation, Dallas, Texas 75234 (US)
(72) Inventor: KOCH, Howard, Frederick, III, Corpus Christi, TX 78410 (US); REED, Randal, P., Corpus Christi, TX 78413 (US); RYAN, Debra, Ann, Corpus Christi, TX 78413 (US)
(74) Representative: James, Anthony Christopher W.P.
(86) International application number: PCT/US2000/032468
(87) International publication number: WO 2001/046111

(56) References cited:
- EP-A- 0 439 013
- EP-A- 0 792 865
- GB-A- 2 059 419

## Description

### TECHNICAL FIELD

This invention relates generally to reaction of gases and liquids and particularly to oxidation reactions. Even more specifically, this invention relates to oxidation of aldehydes to acids where the reaction rate, yield and selectivity are improved.

### BACKGROUND

Oxidation processes for conversion of aldehydes to acids are known. Many documents outline various improvements to the technology.

WO 99/54274 discloses a free-radical process for the production of organic acids from oxo aldehydes (ie., those aldehydes prepared by hydroformylation process) wherein a specific liquid oxidation reactor is employed and the temperature is maintained from about -25°C to about 125°C and kept within 3 degrees of the target. The focus of the invention is on temperature control of the reaction.

EPO 439.013 discloses enhancing reaction of gases and liquids by employing an impeller means to impart shear energy to the liquid and gases and achieve bulk mixing while maintaining the reaction temperature at about -10°C or below. The document discloses oxidation reactions involving free-radical mechanisms. Lowering reaction temperature is said to result in an increase in yield or reaction efficiency, particularly in the case of reaction intermediates such as free-radicals. This document further suggests that reaction rate can be increased by an increase in pressure.

US 5,504,229 discloses the production of C₂-C₂₂ carboxylic acids by oxidation of relevant aldehydes with oxygen in the presence of alkali metal compounds or alkaline metal earth compounds. Distillation and recycle of the bottom product, which contains an alkali metal salt or an alkaline metal earth salt is said to improve the process.

French Publication 2,769,624 discloses the preparation of a carboxylic acid by a process of oxidation with oxygen of the corresponding aldehyde, in the presence of an alkaline or alkaline earth agent at a temperature between 0°C and 25°C. The invention purportedly is particularly suited for the preparation of 2-ethylhexanoic acid. Pressures of between 2 and 7 bar and up to 15 bar are suggested.

There is a commercial need to provide a more efficient process for these oxidation reactions. Specifically the need exists for a faster reaction rate, combined with improved desirable product yields and selectivity.

### SUMMARY

The present invention, in one aspect, provides a gas-liquid process for the oxidation of an organic chemical selected from the group consisting of aldehydes, alcohols, alkylbenzenes and cyclic aliphatic hydrocarbons, comprising:
(a) introducing a gas comprising air, oxygen, or oxygen-enriched or oxygen-containing air into a body of liquid containing the organic chemical in a reaction system in the substantial absence of an added metal catalyst or an added metal free-radical initiator to cause an oxidation reaction between the gas and the organic chemical;
(b) maintaining the reaction system at a temperature in the range of 100°C or less and a pressure of 30 psig (207 kPa gauge) or less during the oxidation reaction; and
(c) mixing the contents of the reaction system to achieve a rate of the oxidation reaction of at least 300 mmol/L/min.

The present invention thus provides an oxidative process for conversion of an aldehyde to an acid at reaction rates exceeding 300 mmol/L/min, wherein the reaction is conducted at moderate temperatures and pressures. In particular, the invention, in another aspect, provides for the production of carboxylic acids and comprises reacting a linear or branched aldehyde having 2 to 22, preferably 4 to 10, carbon atoms with a gas, the gas being air, oxygen, oxygen-enriched air, oxygen-containing air, or combinations thereof in the substantial absence of a metallic free-radical initiator and/or a metallic catalyst. The oxidative process preferably takes place at a temperature in the range of from 20-100°C, and a pressure in the range of from 0-30 psig (0-207 kPa gauge). The oxidative process has a yield and selectivity generally above 90%. One or more organic acids in high purity can be produced via the present invention.

The inventive process further utilizes means for mixing of the liquid and gas mixture. Mixing can be accomplished by mechanical means such as agitation, or nonmechanical means such aeration or sparging, or combinations thereof to improve or maintain the mass transfer between the gas the liquid aldehyde.

### DETAILED DESCRIPTION

As disclosed in EP 439013 by Praxair Technology Inc, a known organic oxidation process carried out in commercial operations is the oxidation of 2-ethylhexanal to 2-ethylhexanoic acid. This process is commonly carried out by sparging air at 120 psig (827.4 kPa gauge) into a bubble column type reactor. The reaction rate, expressed in millimoles of oxygen consumed per liter of liquid per minute (mmol/L/min), is nominally about 104 in the first of a series of reactors in commercial operation. EP 439013 further reports that in the laboratory employing a stirred reactor, rates of 8-14 mmol/L/min were observed. For laboratory experiments employing a bubble reactor, rates of 25-34 mmol/L/min were observed.

The present inventors conducted studies employing equivalent temperatures, but lower pressure conditions, using pure oxygen or air in a 1 liter laboratory reactor (pressure approximately 0.4 psig (2.76 kPa gauge) was employed). Rates in excess of 300 mmol/L/min were observed in these studies for both gases.

While it is known that branched aldehydes oxidize more readily than linear aldehydes, the present invention system is shown to be effective for the oxidation of both linear and branched aldehydes.

It is generally accepted in the art that lower temperatures produce lower byproduct formation (higher selectivity) but with an accompanying decrease in conversion and reaction rate, while the opposite is true of oxidation reactions occurring at higher temperatures. It has been found that the inventive process does not exhibit these limitations, instead producing high yields over a wide range of temperatures with a consistently high reaction rate without the need to begin the oxidation reaction at exceptionally low reaction temperatures or high pressures. Reaction rates observed for the illustrative examples herein were limited merely by the amount of O₂ that could be fed to the reactor. Higher rates are contemplated. Artisans will recognize that this limitation can be overcome by redesign of the plumbing and reaction set up in the laboratory.

The discovery of the invention, wherein increased mass transfer can be employed to achieve enhanced reaction rates and higher yields represents a particularly desirable advance in the art. The invention enables oxidation reactions to operate under simple, low pressure gas-liquid mixing conditions and to be applied more economically and safely.

### Introduction

The present invention provides for a gas-liquid reaction for the oxidation of an aldehyde which involves a free-radical mechanism. The reaction occurs in the substantial absence of a metallic free-radical initiator and/or a metallic catalyst. In particular, disclosed is a gas-liquid process for the oxidation of an organic chemical which involves the presence of alternate reaction pathways leading to undesired byproducts comprising:
introducing air, oxygen, oxygen-enriched. or oxygen-containing air into a body of liquid containing said organic chemical in a reaction system in which means are employed to achieve bulk mixing thereof sufficient so as the rate of reaction is at least 300 mmol/L/min and typically greater than about 500 mmol/L/min.

In the oxidation of an organic chemical, there are generally competing reaction pathways that lead to the formation of unwanted byproducts. To obtain high yields. the reaction leading to the desired product must be able to proceed at a higher rate than undesired side reactions. In a free-radical process, such as the oxidation of aldehydes to carboxylic acids. this is often the case, but often the reactor system is unable to accommodate the high reaction rate leading to the product, thus decreasing the rate of the desired reaction relative to that of the undesired reactions. The present invention accommodates higher rates than previously reported in the art, thus allowing the desired reaction pathway to proceed at a faster rate than the production of unwanted byproducts, thus giving a higher product yield.

For reasons not well understood, it has been discovered that high reaction rates and selectivity of oxidation reactions generally greater than about 90%, can be achieved by the reaction of gas and liquid in a simple reaction system, generally run at about atmospheric pressure and moderate temperatures.

The art is replete with discussions on oxidation of aldehydes wherein the reaction system required to achieve oxidation with the desired results involves use of expensive, complicated reactors. Reactors typically described include bubble column reactors, tube reactors, liquid oxidation reactor, stirred tank reactor, AGR, and the like. The present invention is not reactor limited in the sense that a particular type of reactor is required to achieve the desired result (i.e., high oxidation rates and selectivity). One of skill in the art will appreciate that good mixing of reaction components is necessary along with a mechanism of adequately removing the heat generated during reaction.

While within the scope of the invention to employ any type of reactor system in which shear energy is imparted by the particular mixing mechanism, it is generally preferred to employ relatively high shear energy systems with a substantial volume at high turbulence levels. The particular reactor can be determined for any particular application of course upon consideration of the particular liquid-gas reaction involved and the level of performance necessary or desired in that application.

The discovery of the invention. wherein a moderate temperature and pressure is employed to achieve high reaction rates and selectivity while employing a relatively simple and inexpensive reaction system represents an advance in the art. Not only are moderate temperatures and pressures desirable from an economic and operational viewpoint, it will be appreciated that undesirable intermediate byproduct formation and /or side reactions can often be avoided due to the rapid reaction rate.

The inventive process may be operated under either batch or continuous mode.

Although oxidation of aldehydes to acids are exemplified herein, the inventive process may also be used for other oxidation reaction processes. It has general applicability to the oxidation of various organic chemicals involving reaction intermediates that may tend to decompose at conventional or elevated reaction temperatures or undergo undesired side reactions. The absence of a free-radical initiator or metallic catalyst offers the advantage of cost reduction, ease of production and simplicity of operation. The lower reaction temperature and pressure offer the advantage of cost reduction, reduced safety concerns, and simplification of process design.

To the extent the description is specific, it is solely for the purpose of illustrating preferred embodiments of my invention and should not be taken as limiting the present invention to these specific embodiments.

### Organic Liquids/Aldehydes

Organic liquids which may be employed in the process of this invention include, for example, aldehydes, alcohols, alkylbenzenes, cyclic aliphatic hydrocarbons and the like. Illustrative aldehydes include, for example. formaldehyde, acetaldehyde, propionaldehyde, butyraldehyde, 2-methylbutylaldehyde, iso-butyraldehyde, n-valeraldehyde, caproaldehyde, heptaldehyde, nonylaldehyde, phenylacetaldhyde, and benzaldehyde. Illustrative alcohols include for example. 2-ethylhexanol, 2-propylheptanol, isobutylalcohol, 2-methyl-1-butanol and the like. Illustrative alkylbenzenes include for example, p-nitrotoluene, o-bromotoluene, ethylbenzene, toluene, and the like. Illustrative cyclic aliphatic hydrocarbons include for example, cyclohexane, cyclooctane, cycloheptane and the like. Illustrative organic liquids include those permissible starting materials which are described in Kirk Othmer, Encyclopedia of Chemical Technology, third edition, 1984, the pertinent portions which are incorporated by reference herein.

Preferred organic liquids include aldehydes with any number of carbon atoms. For example, straight chain or branched aldehydes having 2-22, preferably 3-13, and most preferably 4-10 carbon atoms may be employed. Illustrative aldehydes include, for example, decanal. 2-ethyl hexanal, propionaldehyde, butyraldehyde, isobutyraldehyde, n-hexanal, isodecanal, n-heptanal, n-nonanal, isononanal and the like. Mixtures of aldehydes, e.g., linear, branched, and/or oxo-aldehydes, as feed for an oxidation process may also be employed.

### Acids

Embodiments of the present invention include acids known to be produced from the aldehydes described above. In general, acids with any number of carbon atoms may be produced via oxidation in processes described herein. For example, straight chain or branched acids having 2-22 carbon atoms. Exemplary acids include, but are not limited to, 2-ethylhexanoic acid, propanoic, butyric, isobutyric, hexanoic, isononanoic, decanoic, pentadecanoic, isodecanoic , heptanoic and nonanoic acids.

### Metallic Catalysts

In many of the processes used commercially and described in the literature, the oxidation of aldehydes to acids occurs in the presence of sufficient amounts of catalysts or free-radical initiators. Such catalysts or free-radical initiators include, but are not limited to, transition metal salts from such transition metals as manganese, cobalt, titanium, zirconium, iron, nickel and/or copper.

The present invention occurs in the substantial absence of such metallic catalysts or metallic free-radical initiators. By substantial absence, it is meant generally less than 0.5%, preferably 0% of such metal catalysts or metal free-radical initiators.

### Inhibitors

It has been found that the maximization of desired reactions (aldehydes to acids), as described by yield and selectivity, may be enhanced by the use of small quantities of inhibitors. Such inhibitors include, but are not limited to alkali metal salts or alkaline earth metal salts. Bases can also be added for such purposes, such bases include, but are not limited to oxides, hydroxides, acetates, and salts of the carboxylic acids made from the described oxidation reaction. Such materials as sodium hydroxide and or potassium hydroxide are often used. Such inhibitors are useful in inhibiting undesired reactions when present in the reaction at levels generally below 5 wt %, preferably below 3 wt %, more preferably below 2 wt %, based on the total weight of the aldehyde.

Typically, inhibitors were employed in small quantities when oxidizing branched aldheydes. Those of skill in art will recognize when to use inhibitors, which type, and what quantity to employ when oxidizing aldehydes.

### Oxidizing Agent/Gas

Oxidizing gases employed in the present invention include air, oxygen, oxygen enriched air, oxygen containing gases and/or mixtures thereof.

The preferred oxidizing agents useful in the process of this invention are pure oxygen, oxygen enriched or oxygen containing air containing at least 20% oxygen. Preferably, pure oxygen is employed. The oxygen-enriched air may contain for example, inert gases such as nitrogen, carbon dioxide or noble gases. Such oxidizing agents can be used in amounts described herein and in accordance with conventional methods. The agent is employed in an amount sufficient to permit complete oxidation of the organic liquid, eg. aldehyde. Preferably the oxidizing agent is added at a rate suffiicent to suppress or eliminate side reactions of the organic liquid such as decarbonylation of the aldehyde.

### Reaction Pressures

The oxidation reaction of the present invention may be conducted at a reaction pressure of from 0-30 psig (0-207 kPa gauge), preferably in the range of from 2-25 psig (13.7-172.3 kPa gauge) more preferably in the range of from 4-20 psig (27.5-138 kPa gauge), and most preferably less than 4 psig (27.5 kPa gauge). Laboratory experiments were conducted with pressures less than 1 psig (6.9 kPa gauge) and rates of greater than about 300 mmol/L/min were observed. In a commercial setting, it is recognized that greater pressures may be employed to move liquids as opposed to operating the reaction.

### Reaction Vessels

Any standard reactor may be used to attain the reaction rates, selectivity and yield discussed herein, including reactor schemes known to those of ordinary skill in the art. Stainless steel reactors are preferred. Reaction pressure is typically fixed by considerations of vessel wall thickness.

The oxidizing gases can be brought into intimate contact with the aldehyde in any manner that will achieve excellent mixing and high mass transfer sufficient to attain the reaction rates, selectivity and yield outlined herein. Typical reactor schemes include stirrers. axial impellers. turbines, injectors, submerged porous diffusers, spargers, surface aerators, or combinations thereof. Baffles and other mechanisms on or m the reaction vessel can be employed. The materials of construction for the reaction vessel should be inert to the starting reaction components. The fabrication of the vessel should be able to withstand the reaction temperatures and pressures. Means to introduce and/or adjust quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one or more of the starting ingredients. The processes may be conducted in either glass lined, stainless steel, or similar type reaction equipment. The reaction zone may be fitted with one or more internal or external heat exchanger(s) in order to control undue temperture flucations, or to prevent any possible "runaway" reaction temperature.

Stirrers known to those of skill in the art, will include axial impellers, turbines, single stirrers using two or more blades in a single plane, and multiple arrays of blades on a single shaft, with two or more sets of blades in different planes. Multiple shafts rotating in the same or different directions may also be employed.

Combinations of stirring and bubbling or sparging may also be employed, provided the combination provides the reaction rate, yield, and selectivity outlined herein.

An important feature of the invention is to have a reactor which allows for uniformily distributed gas and liquid. Thus in operation, there are essentially no oxygen starved regions due to poor gas-liquid contact.

### Reaction Rate

The aldehyde and gas will react a rate greater than 300 mmol/L/min, typically greater than about 400, most often observed rates exceeded 500 mmol/L/min. Faster rates were contemplated as the rates disclosed and exemplified herein are limited merely by the ability to feed gas to the reactor. Without such a limitation, rates in excess of 550 or 600 mmoles/l/min. are expected.

Those of ordinary skill in the art will appreciate that high conversion rates (difference between the amount of aldehyde fed to the reactor and the amount of substantially unreacted aldehyde present in the reaction system post-reaction) can be obtained variously by increasing the temperature, increasing the pressure, and combinations thereof. However, conversion, while important, and desireably as high as possible, does not tell the whole story. High conversions without a high selectivity (providing the maximum amount of desireable conversion product) and/or high yields generally do not meet the needs of commercial producers of acids.

### Yield

Yield (of the desired acid) is defined in the present context as the product of conversion and selectivity. The yields of desired product by methods described herein have been found to be generally greater than about 80%, and typically greater than about 90%. Yield can be somewhat different for linear versus branched aldehydes and was observed to be at least 96% in the experiments conducted.

### Selectivity

Selectivity is defined as the amount of desired product, generally the acid corresponding to the fed aldehyde, divided by the amount of fed aldehyde reacted, expressed as a percent. Selectivity observed in the present invention was generally greater than 85%. Selectivity, as in the yield discussed above, can be different for branched versus linear aldehydes. For linear aldehydes, typical selectivity values observed were greater than 95% and usually above 98%. For branched aldehdyes, typical selectivity values observed were greater than about 90%, and usually above 95%.

### Reaction Temperatures

Reaction temperatures in embodiments of the invention will generally be below 100°C, preferably below 70°C. Typical laboratory, batch reaction temperatures were begun at about room temperature and quickly ramped to 50 to 90°C. Useful temperatures in embodiments of the invention include those in the range of from 20-100 °C, preferably 30-80, more preferably 50-70°C.

### EXAMPLES

In accordance with the invention, various runs were conducted comparing rates of linear and branched aldehydes employing pure oxygen as well as mixtures thereof. The laboratory reactor employed consisted of a 6 inch (152.4 mm) section of glass pipe capped with stainless steel plates on each end. Gas was fed from the bottom of the reactor through a 1/8" (3.18 mm) stainless steel tube. Aldehyde was fed in from the top plate through a 1/2" (12.7 mm) stainless steel tube. The reactor further consisted of a gas outlet and a combination heating/cooling coil. A stainless steel stirrer having a six bladed impeller, approximately 1 1/2 inches (38.1 mm) in diameter affixed to the top plate and able to rotate up to 3000 rpm was used. Temperature was measured through a thermocouple attached to the bottom plate.

All runs were conducted at about atmospheric pressure. Reaction pressure was monitored and did not rise above 0.5 psig (3.45 kPa gauge). In each of the examples, 800 ml of aldehyde was fed to the reactor, and the oxidizing gas was fed at a constant rate of about 5 liters/minute. Unless so noted, pure oxygen was used as the oxidizing gas.

The aldehyde and gas were contacted as noted above and stirred for the period as stated in Table 1. Samples were taken at appropriate intervals to monitor reaction progress through gas chromotography analysis. Runs were conducted at from about 1 to less than 3 hours. Table 1 identifies reaction conditions and results of both linear and branched aldehyde conversions.

### Branched Aldehyde: 2-Ethylhexanal Conversion

An inhibitor was used when studying branched aldehydes. Amounts employed are as stated in Table 1.

Gas chromatographic analysis of the product from the laboratory tests show that the yield of product acid is higher in these laboratory tests than in commercial practice, probably due to a decrease in the amount of by-products formed.

While it is known that branched aldehydes oxidize more readily than linear aldehydes, the present system is shown to be effective for the oxidation of linear aldehydes as well.

**Table 1:**

| **Aldehyde Oxidation in a 1 L Reactor System** | | | | | | |
|---|---|---|---|---|---|---|
| **C9 Runs** | **Temp (°C)** | **Time (hrs)** | **Conv (%)** | **Selec (%)** | **Yield (%)** | **Rate (mmol/L/min)** |
| 1 | 65 | 1.27 | 98.2 | 98.7 | 96.9 | 500 |
| 2 | 70 | 1.52 | 98.2 | 99 | 97.2 | 500 |
| 3 | 50 | 1.53 | 97.7 | 100 | 97.7 | 500 |
| 4 | 50 | 1.65 | 97.3 | 99.9 | 97.2 | 500 |
| 5 | 55 | 1.57 | 97.5 | 99.7 | 97.2 | 500 |
| 6 | 55 | 1.8 | 98.4 | 99.7 | 98 | 500 |
| 7 | 47 | 2.42 | 96.8 | 99.9 | 96.7 | 500 |
| 8 r | 59 | 2.27 | 97.2 | 100 | 97.3 | 500 |
| 9 a | 50 | 2.97 | 97.3 | 98.9 | 96.2 | 500 |
| 10 | 68 | 1.5 | 98.2 | 99.2 | 97.4 | 500 |
| 11 | 65 | 1.67 | 98.6 | 99.2 | 97.8 | 500 |

| **C7 Runs** | | | | | | |
|---|---|---|---|---|---|---|
| 12 b | 65 | 1 | 98.5 | 97.8 | 96.3 | 500 |
| 13 b | 64 | 1 | 98 | 97.9 | 96 | 500 |
| 14 b | 65 | 1.5 | 98.5 | 98.2 | 96.8 | 500 |
| 15 b | 66 | 1.5 | 98.2 | 98.4 | 96.6 | 500 |
| 16 b | 68 | 1.5 | 97.8 | 98.4 | 96.2 | 500 |
| 17 | 67 | 1.5 | 98.4 | 99.2 | 97.7 | 500 |
| 18 | 68 | 1.5 | 99.6 | 98.8 | 98.4 | 500 |
| 19 | 68 | 1.5 | 99.9 | 97.8 | 97.8 | 500 |
| 20 | 68 | 1.42 | 99.8 | 98.7 | 98.5 | 500 |
| 21 | 68 | 1 | 99.2 | 99.1 | 98.2 | 500 |
| 22 | 67 | 1 | 98.7 | 99.5 | 98.2 | 500 |
| 23 | 67 | 1.33 | 97.1 | 99.8 | 96.8 | 500 |
| 24 | 67 | 1.5 | 98.8 | 99.5 | 98.3 | 500 |
| 25 | 57 | 1.75 | 95.8 | 99.8 | 95.6 | 500 |
| 26 | 75 | 1 | 98 | 99.1 | 97.1 | 500 |

| **2-Ethylhexanal Runs** | | | | | | |
|---|---|---|---|---|---|---|
| 27 | 68 | 1 | 99.9 | 77.7 | 77.6 | 500 |
| 28 | 55 | 1 | 99.8 | 74.9 | 74.8 | 500 |
| 29 | 48 | 0.83 | 99.6 | 75.2 | 74.9 | 500 |
| 30 | 49 | 0.83 | 100 | 76.4 | 76.4 | 500 |
| 31 | 40 | 1 | 99.1 | 80.1 | 79.4 | 500 |
| 32 | 40 | 1 | 99.7 | 79.2 | 78.9 | 500 |
| 33 x | 60 | 0.83 | 100 | 95.1 | 95.1 | 500 |
| 34 y | 60 | 0.83 | 100 | 96.4 | 96.4 | 500 |
| x = 1 mol% Potassium 2-ethylhexanoate salt | | | | | | |
| y=1.6 mol% Potassium 2-ethylhexanoate salt | | | | | | |
| a=air | | | | | | |
| r=2000 rpm | | | | | | |
| b=high branched content | | | | | | |

### CONCLUSION

As can be seen from the data in Table 1, rates above 300mmol/L/min, and in fact about 500 mmol/L/min were observed utilizing a simple reactor setup and design in the absence of a metallic catalyst. Although the art has discussed oxidation reactions, there was no discussion or suggestion found in the art that rates in excess of 300 mmol/L/min utilizing moderate temperatures and pressures could be achieved. The present invention illustrates this point.

In addition to utilization of stirred reaction systems, experiments were also conducted in the absence of the stirrer and rates were continued to be observed at greater than about 300 mmol/L/min. This indicates that reactor design and use of the stirrer mechanism are not the basis for the high rates observed herein.

### Branched Aldehyde: 2-Ethylhexanal Conversion

Per the art (EP 0 792 865) product yield in commercial practice for 2-EH is about 85%, with close to 100% conversion. The yield obtained in laboratory tests for 2-EH was found to be about 95%, with about 95% conversion.

### Linear Aldehyde Conversion

For aldehyde conversion the yield obtained in laboratory tests was 98% with up to 100% selectivity.

## Claims

1. A gas-liquid process for the oxidation of an organic chemical selected from the group consisting of aldehydes, alcohols, alkylbenzenes and cyclic aliphatic hydrocarbons, comprising:
(a) introducing a gas comprising air, oxygen, or oxygen-enriched or oxygen-containing air into a body of liquid containing the organic chemical in a reaction system in the substantial absence of an added metal catalyst or an added metal free-radical initiator to cause an oxidation reaction between the gas and the organic chemical;
(b) maintaining the reaction system at a temperature in the range of 100°C or less and a pressure of 30 psig (207 kPa gauge ) or less during the oxidation reaction; and
(c) mixing the contents of the reaction system to achieve a rate of the oxidation reaction of at least 300 mmol/L/min.

2. The process of claim 1 in which the said organic chemical is selected from linear and branched aldehydes having 2 to 22 carbon atoms and combinations thereof, which process comprises, as step (b),
maintaining the reaction system at a temperature in the range of 20-100°C and a pressure in the range of 0 to 30 psig (0 to 207 kPa gauge) during the oxidation reaction.

3. The process of claim 1 wherein said gas is oxygen.

4. The process of claim 2 wherein said gas is oxygen.

5. The process of claim 2 wherein the reaction temperature at the beginning of the reaction is room temperature and the temperature is increased to 50°C and further to 90°C.

6. The process of claim 1 or 2 wherein the reaction temperature is in the range of 30°C to 80°C.

7. The process of claim 1 or 2 wherein the reaction temperature is in the range of 50°C to 70°C.

8. The process of claim 1 wherein the reaction pressure is in the range of 2 to 25 psig (13.7 to 172.3 kPa gauge).

9. The process of claim 8 wherein the reaction pressure is in the range of 4 to 20 psig (27.5 to 137.9 kPa gauge).

10. The process of claim 1 or 8 wherein the reaction pressure is less than 4 psig (27.5 kPa gauge).

11. The process of claim 2 wherein the reaction pressure is in the range of 2 to 25 psig (13.7 to 172.3 kPa gauge).

12. The process of claim 11 wherein the reaction pressure is in the range of 4 to 20 psig (27.5 to 137.9 kPa gauge).

13. The process of claim 2 or 11 wherein the reaction pressure is less than 4 psig (27.5 kPa gauge).

14. The process of claim 2 wherein oxygen is contacted with n-heptanal at a temperature of about 60°C, at about atmospheric pressure, and wherein the oxidation reaction rate is at least 500 mmol/L/min.

15. The process of claim 2 wherein oxygen is contacted with n-nonanal at a temperature of about 50°C, at about atmospheric pressure, and wherein the oxidation reaction rate is at least 500 mmol/L/min.

16. The process of claim 2 wherein oxygen is contacted with n-ethylhexanal at a temperature of about 50°C, at atmospheric pressure, and wherein the oxidation reaction rate is at least 500 mmol/L/min.

## Patentansprüche

1. Gas-Flüssigkeits-Verfahren für die Oxidation einer organischen Chemikalie, die aus der Gruppe ausgewählt ist, bestehend aus Aldehyden, Alkoholen, Alkylbenzolen und cyclischen aliphatischen Kohlenwasserstoffen, umfassend:
(a) das Einführen eines Gases, das Luft, Sauerstoff oder Sauerstoffangereicherte oder Sauerstoff-enthaltende Luft umfasst, in den Körper einer Flüssigkeit, die die organische Chemikalie enthält, in einem Reaktionssystem bei weitgehender Abwesenheit eines zugefügten Metall-Katalysators oder eines zugefügten metallfreien, radikalischen Initiators, um eine Oxidationsreaktion zwischen dem Gas und der organischen Chemikalie zu bewirken,
(b) das Halten des Reaktionssystems bei einer Temperatur im Bereich von 100 °C oder weniger und einem Druck von 30 psig (207 kPa Manometerdruck) oder weniger während der Oxidationsreaktion, und
(c) das Vermischen der Inhaltsstoffe des Reaktionssystems, um eine Oxidationsreaktionsrate von wenigstens 300 mmol/l pro min zu erreichen.

2. Verfahren gemäß Anspruch 1, in dem die organische Chemikalie aus linearen und verzweigten Aldehyden mit 2 bis 22 Kohlenstoffatomen und Kombinationen derselben ausgewählt ist, wobei das Verfahren als Schritt (b) das Halten des Reaktionssystems bei einer Temperatur im Bereich von 20 bis 100 °C und einem Druck im Bereich von 0 bis 30 psig (0 bis 207 kPa Manometerdruck) während der Oxidationsreaktion umfasst.

3. Verfahren gemäß Anspruch 1, wobei das Gas Sauerstoff ist.

4. Verfahren gemäß Anspruch 2, wobei das Gas Sauerstoff ist.

5. Verfahren gemäß Anspruch 2, wobei die Reaktionstemperatur zu Beginn der Reaktion Raumtemperatur ist, und die Temperatur auf 50 °C erhöht wird und weiterhin auf 90 °C erhöht wird.

6. Verfahren gemäß den Ansprüchen 1 oder 2, wobei die Reaktionstemperatur im Bereich von 30 °C bis 80 °C liegt.

7. Verfahren gemäß den Ansprüchen 1 oder 2, wobei die Reaktionstemperatur im Bereich von 50 °C bis 70 °C liegt.

8. Verfahren gemäß Anspruch 1, wobei der Reaktionsdruck im Bereich von 2 bis 25 psig (13,7 - 172,3 kPa Manometerdruck) liegt.

9. Verfahren gemäß Anspruch 8, wobei der Reaktionsdruck im Bereich von 4 bis 20 psig (27,5 - 137,9 kPa Manometerdruck) liegt.

10. Verfahren gemäß den Ansprüchen 1 oder 8, wobei der Reaktionsdruck geringer als 4 psig (27,5 kPa Manometerdruck) ist.

11. Verfahren gemäß Anspruch 2, wobei der Reaktionsdruck im Bereich von 2 bis 25 psig (13,7 - 172,3 kPa Manometerdruck) liegt.

12. Verfahren gemäß Anspruch 11, wobei der Reaktionsdruck im Bereich von 4 bis 20 psig (27,5 - 137,9 kPa Manometerdruck) liegt.

13. Verfahren gemäß den Ansprüchen 2 oder 11, wobei der Reaktionsdruck geringer als 4 psig (27,5 kPa Manometerdruck) ist.

14. Verfahren gemäß Anspruch 2, wobei Sauerstoff bei einer Temperatur von etwa 60 °C und etwa atmosphärischem Druck mit n-Heptanal in Kontakt gebracht wird, und wobei die Oxidationsreaktionsrate wenigstens 500 mmol/l pro min ist.

15. Verfahren gemäß Anspruch 2, wobei Sauerstoff bei einer Temperatur von etwa 50 °C und etwa atmosphärischem Druck mit n-Nonanal in Kontakt gebracht wird, und wobei die Oxidationsreaktionsrate wenigstens 500 mmol/l pro min ist.

16. Verfahren gemäß Anspruch 2, wobei Sauerstoff bei einer Temperatur von etwa 50 °C und etwa atmosphärischem Druck mit n-Ethylhexanal in Kontakt gebracht wird, und wobei die Oxidationsreaktionsrate wenigstens 500 mmol/l pro min ist.

## Revendications

1. Procédé gaz-liquide pour l'oxydation d'un produit chimique organique choisi dans le groupe formé par les aldéhydes, les alcools, les alkylbenzènes et les hydrocarbures cycloaliphatiques, comprenant :
(a) l'introduction d'un gaz comprenant de l'air, de l'oxygène ou de l'air enrichi en oxygène ou contenant de l'oxygène dans une masse de liquide contenant le produit chimique organique dans un système réactionnel en l'absence substantielle de catalyseur métallique ajouté ou d'initiateur radicalaire métallique ajouté, pour provoquer une réaction d'oxydation entre le gaz et le produit chimique organique ;
(b) le maintien du système réactionnel à une température comprise dans l'intervalle de 100°C ou moins et à une pression de 30 psig (207 kPa au manomètre) ou moins pendant la réaction d'oxydation ; et
(c) le mélange du contenu du système réactionnel pour atteindre une vitesse de la réaction d'oxydation d'au moins 300 mmol/l/min.

2. Procédé selon la revendication 1, dans lequel ledit produit chimique organique est choisi parmi les aldéhydes linéaires et ramifiés ayant 2 à 22 atomes de carbone et leurs associations, lequel procédé comprend, comme étape (b),
le maintien du système réactionnel à une température comprise dans l'intervalle de 20 à 100°C et à une pression comprise dans l'intervalle de 0 à 30 psig (0 à 207 kPa au manomètre) pendant la réaction d'oxydation.

3. Procédé selon la revendication 1, dans lequel ledit gaz est l'oxygène.

4. Procédé selon la revendication 2, dans lequel ledit gaz est l'oxygène.

5. Procédé selon la revendication 2, dans lequel la température réactionnelle au début de la réaction est la température ambiante et la température est élevée jusqu'à 50°C, puis jusqu'à 90°C.

6. Procédé selon la revendication 1 ou 2, dans lequel la température réactionnelle est comprise dans l'intervalle de 30°C à 80°C.

7. Procédé selon la revendication 1 ou 2, dans lequel la température réactionnelle est comprise dans l'intervalle de 50°C à 70°C.

8. Procédé selon la revendication 1, dans lequel la pression réactionnelle est comprise dans l'intervalle de 2 à 25 psig (13,7 à 172,3 kPa au manomètre).

9. Procédé selon la revendication 8, dans lequel la pression réactionnelle est comprise dans l'intervalle de 4 à 20 psig (27,5 à 137,9 kPa au manomètre).

10. Procédé selon la revendication 1 ou 8, dans lequel la pression réactionnelle est inférieure à 4 psig (27,5 kPa au manomètre).

11. Procédé selon la revendication 2, dans lequel la pression réactionnelle est comprise dans l'intervalle de 2 à 25 psig (13,7 à 172,3 kPa au manomètre).

12. Procédé selon la revendication 11, dans lequel la pression réactionnelle est comprise dans l'intervalle de 4 à 20 psig (27,5 à 137,9 kPa au manomètre).

13. Procédé selon la revendication 2 ou 11, dans lequel la pression réactionnelle est inférieure à 4 psig (27,5 kPa au manomètre).

14. Procédé selon la revendication 2, dans lequel de l'oxygène est mis en contact avec du n-heptanal à une température d'environ 60°C, au voisinage de la pression atmosphérique, et dans lequel la vitesse de la réaction d'oxydation est d'au moins 500 mmol/l/min.

15. Procédé selon la revendication 2, dans lequel de l'oxygène est mis en contact avec du n-nonanal à une température d'environ 50°C, au voisinage de la pression atmosphérique, et dans lequel la vitesse de la réaction d'oxydation est d'au moins 500 mmol/l/min.

16. Procédé selon la revendication 2, dans lequel de l'oxygène est mis en contact avec du n-éthylhexanal à une température d'environ 50°C, à la pression atmosphérique, et dans lequel la vitesse de la réaction d'oxydation est d'au moins 500 mmol/l/min.
